# DEMANDE DE BREVET EUROPEEN

(11) **EP 3 170 448 A1**
(43) Date de publication de la demande: **24.05.2017**
(21) Numéro de dépôt: 16199326.6
(22) Date de dépôt: 17.11.2016
(51) Int. Cl.: A61B 5/02, A61B 5/021, A61B 5/024

(54) **DISPOSITIF ET PROCÉDÉ DE MESURE DE LA RIGIDITÉ ARTÉRIELLE D'UN PATIENT**

(30) Priorité: 17.11.2015 FR 1561051
(71) Demandeur: BMM INVEST S.A., 5326 Contern (LU)
(72) Inventeur: CONSTANT, Jonathan, 34000 Montpellier (FR); COCHARD, Etienne, 34670 Baillargues (FR)
(74) Mandataire: Delaveau, Sophie

(57) **Abrégé**

L'invention propose un dispositif fiable, économique et pratique de mesure de vitesse d'onde de pouls et de calcul de la rigidité artérielle.

Le dispositif selon l'invention comprend :
• deux capteurs (100-200) destiné à être positionné, en position d'utilisation, contre la peau d'un patient située sur une artère carotide sur une artère fémorale et comprenant chacun au moins une diode électroluminescente formant source lumineuse et émettant à une longueur d'onde comprise entre 600 et 1400 nm et un convertisseur lumière/fréquence ;
• une unité de commande (300) adaptée pour commander un fonctionnement simultané des deux capteurs (100-200), et pour enregistrer les données envoyées par les deux capteurs ;
• une unité de calcul (400) comprenant une interface d'entrée de la distance (dL) séparant le premier et le deuxième capteur en position d'utilisation, et programmée pour :
∘ Mesurer la différence de temps (dT) entre l'impulsion mesurée au niveau de l'artère carotide et l'impulsion mesurée au niveau de l'artère fémorale
∘ Calculer la rigidité artérielle comme étant égale à 0,8 x d L/dT.

## Description

L'invention concerne un dispositif et un procédé de mesure de la rigidité artérielle du patient.

L'hypertension artérielle est la première maladie chronique dans le monde.

L'un des nombreux facteurs favorisant l'hypertension artérielle est la rigidité artérielle. Elle représente la capacité qu'ont les gros troncs artériels (GTA) proximaux d'amortir la pulsatilité artérielle qui existe au niveau de l'aorte ascendante et de la transformer en un débit continu au niveau des artérioles, de manière à protéger les organes cibles de l'hyperpulsatilité.

Lorsque la rigidité artérielle dépasse des valeurs seuil (dépendant de l'âge du patient), cela peut être le signe que la fonction d'amortissement des GTA est altérée, ce qui entraîne une élévation inappropriée de la pression systolique et diastolique.

Cependant, la connaissance de la rigidité artérielle n'est pas suffisante en soi pour permettre un diagnostic à des fins curatives. De nombreux autres facteurs entrent en ligne de compte, tels que l'âge du patient, son état général, son activité sportive, sa génétique, etc.

La rigidité artérielle peut être calculée à partir de différents types de mesures :
- des mesures indirectes, comme la pression systolique ou pulsée carotidienne et de l'index d'augmentation (Alx = pression d'augmentation/pression pulsée = P2/(P1-P3)). L'avantage de ces dernières mesures est de renseigner sur la charge tensionnelle systolique ou pulsée qui s'exerce sur les organes cibles ; ou
- des mesures directes, comme la mesure de la vitesse de propagation d'une onde de pouls entre deux artères.

La présente invention concerne le calcul de la rigidité artérielle par la mesure de la vitesse de propagation d'une onde de pouls entre deux artères :
- entre l'artère carotide et l'artère fémorale : c'est la vitesse d'onde de pouls (VOP) carotido-fémorale ; ou
- entre l'artère humérale et l'artère tibiale postérieure : c'est la VOP bras-cheville. Cette mesure a été développée dans certaines régions du monde pour éviter d'exposer la région de l'aine. Cependant, la réalité anatomique du trajet parcouru par l'onde de pouls n'est toujours pas déterminée, de sorte que la mesure carotido-fémorale est préférée.

Ainsi, on mesure le temps de transit de l'onde de pouls qui va d'une artère à l'autre. La rigidité artérielle est alors calculée sous la forme d'une vitesse en mètre par seconde grâce à la formule V = D/T où D est la distance carotido-fémorale mesurée chez le patient et T le temps de transit de l'onde de pouls.

Il existe actuellement différentes techniques pour calculer la vitesse de l'onde de pouls : la tonométrie, le doppler, les manchons gonflables, les capteurs mécaniques (effet Hall, piezzo, etc.), l'ECG, l'IRM, etc.

Ces méthodes souffrent de plusieurs inconvénients. Certaines sont très coûteuses et complexes à mettre en oeuvre (IRM, ECG). D'autres nécessitent une bonne expérience de l'opérateur et des capteurs de bonne qualité pour obtenir des mesures fiables et reproductibles.

En outre, pour la mesure de la VOP carotido-fémorale, il est nécessaire d'exposer la région de l'aine pour positionner et fixer correctement le capteur. Outre certaines cultures qui refusent une telle exposition, même à des médecins, cela peut s'avérer gênant chez certains patients et le stress anormalement élevé lors de la mesure peut fausser les résultats.

Enfin, les techniques les plus facile à mettre en oeuvre, que sont la tonométrie et les capteurs de pression sont souvent inefficaces chez les patients qui présentent un indice de masse corporelle élevé. Or, ces personnes sont particulièrement exposées au risque d'hypertension artérielle.

La présente invention vise donc à proposer un dispositif fiable, économique et pratique de mesure de vitesse d'onde de pouls et de calcul de la rigidité artérielle.

Ainsi, l'invention propose d'utiliser deux capteurs utilisant le principe de la photopléthysmographie optique, dérivé de celui utilisé pour mesurer la variation du volume sanguin capillaire. Dans cette application, un seul capteur est utilisé et positionné à l'extrémité du doigt. Au contraire, l'invention utilise deux capteurs photopléthysmographiques de manière synchrone et simultanée, positionnés, en utilisation, sur la peau d'un patient, au-dessus de l'artère carotidienne et de l'artère fémorale.

À cette fin, l'invention a pour objet un dispositif de calcul de rigidité artérielle d'un patient, humain ou animal comprenant :
- un premier capteur destiné à être positionné, en position d'utilisation, contre la peau d'un patient située sur une artère carotide, et comprenant au moins une diode électroluminescente formant source lumineuse et émettant à une longueur d'onde comprise entre 600 et 1400 nm, et un convertisseur lumière/fréquence ;
- un second capteur destiné à être positionné, en position d'utilisation, contre la peau d'un patient située sur une artère fémorale, et comprenant au moins une diode électroluminescente formant source lumineuse (10) et émettant à une longueur d'onde comprise entre 600 et 1400 nm, et un convertisseur lumière/fréquence ; et
- une unité de commande reliée aux capteurs par liaison filaire ou hertzienne, adaptée pour :
   ∘ commander un fonctionnement simultané de la source lumineuse des deux capteurs, et pour
   ∘ enregistrer des données envoyées par le convertisseur des deux capteurs en réponse à un signal lumineux envoyé par les sources lumineuses et réfléchi respectivement par les artères carotide et fémorale ;
- une unité de calcul de la rigidité artérielle comprenant une interface d'entrée de la distance séparant le premier capteur et le deuxième capteur en position d'utilisation, et programmée pour :
   ∘ mesurer la différence de temps entre l'impulsion mesurée au niveau de l'artère carotide et l'impulsion mesurée au niveau de l'artère fémorale
   ∘ calculer la rigidité artérielle comme étant égale à 0,8 x dL/dT, où :
      ▪ dL est la distance séparant le premier et le deuxième capteur en position d'utilisation, et
      ▪ dT est la différence de temps entre l'impulsion mesurée par le premier capteur au niveau de l'artère carotide et l'impulsion mesurée par le second capteur au niveau de l'artère fémorale.

Selon des modes de réalisation particuliers :
- l'unité de commande (300) est adaptée pour commander une illumination en continu de la source lumineuse (10) des deux capteurs (100,200), et un ajustement de l'intensité d'illumination de la source lumineuse (10) des deux capteurs (100,200) selon la quantité de lumière reçue par le convertisseur lumière/fréquence (20) concerné.
- chaque source lumineuse peut être constituée d'une pluralité de diodes électroluminescentes, avantageusement agencées autour du convertisseur lumière/fréquence ;
- chaque source lumineuse peut être suffisante pour que le convertisseur reçoive une irradiance en µW/cm² comprise entre 20 µW/cm² et 500 µW/cm² après réflexion de la lumière par les artères carotide et fémorale ;
- le premier capteur peut comporter un collier réglable de fixation du capteur autour du cou du patient, en position d'utilisation ;
- le second capteur peut être agencé à une extrémité d'un manche de préhension
- le manchon présente une longueur minimum de 15 cm, de préférence une longueur comprise entre 15 et 50 cm ;
- les capteurs peuvent comporter un moyen de mesure de la distance qui les sépare en position d'utilisation ;
- les diodes électroluminescentes infrarouges émettent à une longueur d'onde de 850 nm;
- l'unité de calcul peut comprendre un moyen de réglage d'une fréquence d'échantillonnage comprise entre 100 mesures par seconde et 1000 mesures par seconde, de préférence de 500 mesures par seconde ; et/ou
- l'unité de calcul peut être programmée pour effectuer un filtrage passe-bande entre 0,5Hz et 20Hz avant de mesurer la différence de temps entre l'impulsion mesurée par le premier capteur au niveau de l'artère carotide et l'impulsion mesurée par le second capteur au niveau de l'artère fémorale.

L'invention a également pour objet un procédé de calcul de la rigidité artérielle d'un patient comprenant les étapes suivantes :
(a) positionner un premier capteur comportant au moins une diode électroluminescente formant source lumineuse et émettant à une longueur d'onde comprise entre 600 et 1400 nm ,et un convertisseur lumière/fréquence, contre la peau d'un patient située sur une artère carotide ;
(b) positionner un second capteur comprenant au moins une diode électroluminescente formant source lumineuse et émettant à une longueur d'onde comprise entre 600 et 1400 nm, et un convertisseur lumière/fréquence, contre la peau d'un patient située sur une artère fémorale ;
(c) mesurer la distance séparant le premier et le deuxième capteur en position d'utilisation
(d) actionner les capteurs de manière synchrone et simultanée de manière à mesurer l'onde de pouls entre l'artère carotide et l'artère fémorale ;
(g) mesurer la différence de temps entre l'impulsion mesurée au niveau de l'artère carotide et l'impulsion mesurée au niveau de l'artère fémorale ;
(h) calculer la rigidité artérielle comme étant égale à 0,8 x dL/dT, où :
   ▪ dL est la distance séparant le premier et le deuxième capteur en position d'utilisation, et
   ▪ dT est la différence de temps entre l'impulsion mesurée par le premier capteur au niveau de l'artère carotide et l'impulsion mesurée par le second capteur au niveau de l'artère fémorale.

Selon des modes de réalisation particuliers :
- le procédé peut comprendre, entre les étapes (d) et (g), les étapes consistant à :
   (e) définir une fréquence d'échantillonnage comprise entre 100 mesures par seconde et 1000 mesures par seconde, de préférence de 500 mesures par seconde ;
   (f) effectuer un filtrage passe-bande entre 0,5Hz et 20Hz.
- l'étape (g) de mesure de la différence de temps entre l'impulsion mesurée au niveau de l'artère carotide et l'impulsion mesurée au niveau de l'artère fémorale peut être effectuée par la méthode des tangentes.

L'invention porte enfin sur l'utilisation du dispositif tel que précédemment défini pour appréhender un dysfonctionnement cardio-vasculaire.

D'autres caractéristiques de l'invention seront énoncées dans la description détaillée ci-après, faite en référence aux dessins annexés, qui représentent, respectivement :
- la figure 1, une vue schématique en coupe d'un capteur selon l'invention, placé au-dessus d'une artère ;
- la figure 2, une vue schématique en perspective du dispositif de mesure de vitesse d'onde de pouls selon l'invention en position d'utilisation sur un patient ;
- les figures 3 à 5, des vues schématiques en perspective d'un mode de réalisation préféré d'un capteur photopléthysmographique selon l'invention destiné à être positionné au-dessus de l'artère carotide ;
- la figure 6, une vue schématique en plan du capteur des figures 2 à 4 illustrant l'agencement des sources lumineuses et du convertisseur lumière/fréquence ;
- les figures 7 à 9, des vues schématiques en perspective d'un mode de réalisation préféré d'un capteur photopléthysmographique selon l'invention destiné à être positionné au-dessus de l'artère fémorale ;
- la figure 10, une vue schématique en plan d'un exemple d'interface affichant le signal capté et traité selon une première méthode par le dispositif selon l'invention et le calcul de la rigidité artérielle ; et
- la figure 11, une vue schématique d'une deuxième méthode de calcul de la rigidité artérielle par le dispositif selon l'invention.

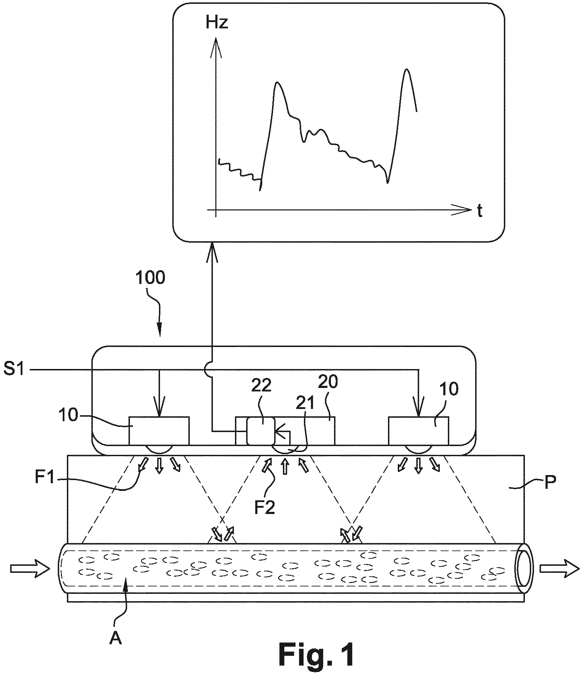

La figure 1 illustre un capteur photopléthysmographique 100 placé contre la peau P d'un patient située sur une artère carotide ou fémorale A.

Le principe de photopléthysmographie repose sur l'absorption de la lumière par l'oxygène présent dans le sang. La lumière émise par le capteur se réfléchit sur un tissu possédant une vascularisation artérielle puis elle est captée par un photodétecteur (dans l'invention, il s'agit d'un convertisseur lumière/fréquence). Le signal obtenu est une onde photopléthysmographique, rythmée par la pulsatilité artérielle et dont l'amplitude dépend de l'absorption lumineuse par le sang artériel. Ce principe permet, dans l'état de la technique, de mesurer les variations du flux sanguin. Selon l'invention, ce principe de photopléthysmographie est utilisé pour évaluer la vitesse de l'onde de pouls.

Le capteur 100 comporte des diodes électroluminescentes 10 émettant de la lumière dans le sens des flèches F1 vers l'artère A. De préférence, les diodes émettent dans une gamme de longueur d'onde allant du rouge à l'infrarouge, soit une longueur d'onde comprise entre 600 et 1400 nm. Selon l'invention, la longueur d'onde des diodes est avantageusement de 850nm, longueur d'onde pour laquelle la lumière pénètre profondément dans la peau et est réfléchie de manière optimale par le sang, ce qui améliore la qualité du signal reçu par le convertisseur lumière/fréquence en rendant plus nette l'allure de la courbe de l'onde de pouls et en optimisant son traitement tel qu'il sera décrit plus loin. L'utilisation d'une longueur d'onde de 850 nm permet ainsi d'obtenir une bonne précision de la vitesse de l'onde de pouls.. Par exemple, des telles diodes sont vendues par la société OSRAM ® sont la référence SFH 4356P Infrared Emitter (850 nm).

Lorsqu'un signal de commande S1 des diodes 10 est envoyé, la lumière est émise vers l'artère. Une partie est alors réfléchie dans le sens des flèches F2 vers le photodétecteur 20. Ce dernier est un convertisseur lumière/fréquence comprenant une photodiode 21 couplée à un convertisseur courant/fréquence 22.La lumière reçue est alors transformée par la photodiode 21 en courant puis convertie en un signal de fréquence par le convertisseur 22. Ce dernier est relié à un enregistreur des données envoyées par le convertisseur du capteur. La conversion lumière fréquence est effectuée sur le capteur, ce qui permet de s'affranchir de toute liaison filaire qui entrainerait une baisse de la qualité du signal.

Par exemple, un tel convertisseur lumière/fréquence est commercialisé par la société Texas Instruments® sous la référence TSL235LF.

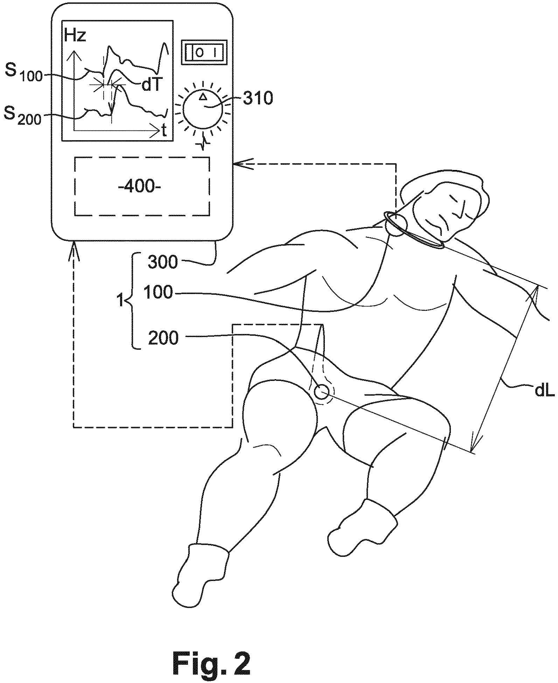

La mise en oeuvre du dispositif selon l'invention est illustré en figure 2.

Selon l'invention, le dispositif 1 de mesure de vitesse d'onde de pouls d'un patient comporte deux capteurs identiques :
- un premier capteur 100 destiné à être positionné, en position d'utilisation, contre la peau d'un patient située sur une artère carotide (voir figure 2), et comprenant au moins une diode électroluminescente formant source lumineuse et émettant à une longueur d'onde comprise entre 600 et 1400 nm, et un convertisseur lumière/fréquence ;
- un second capteur 200 destiné à être positionné, en position d'utilisation, contre la peau d'un patient située sur une artère fémorale (voir figure 2), et comprenant au moins une diode électroluminescente formant source lumineuse et émettant à une longueur d'onde comprise entre 600 et 1400 nm, et un convertisseur lumière/fréquence ; et

Il comporte également une unité de commande 300 reliée aux capteurs 100 et 200 par liaison filaire ou hertzienne.

L'unité de commande 300 est adaptée pour commander un fonctionnement simultané de la source lumineuse 10 des deux capteurs 100 et 200, et pour enregistrer les données S100 et S200 envoyées par les deux capteurs en réponse à un signal lumineux envoyé par les sources lumineuses 10 et réfléchi respectivement par les artères carotide et fémorale.

Le fonctionnement simultané de la source lumineuse 10 des deux capteurs 100 et 200 est assuré dans l'unité de commande. Chaque source lumineuse 10 de chacun des capteurs 100 et 200 est pilotée indépendamment par l'unité de commande grâce à deux convertisseurs numérique-analogique suivis de deux amplificateurs opérationnels, ce qui assure le contrôle de l'intensité lumineuse des diodes électroluminescentes des deux capteurs 100,200. Les diodes sont contrôlées en illumination continue, ce qui permet de ne pas perturber le signal reçu par le capteur et d'optimiser la qualité du signal à traiter. Dans le cadre de cette illumination en continu, la variation de l'intensité d'illumination est ajustée selon le degré de pénétration de la lumière dans la peau. Si le convertisseur lumière/fréquence reçoit trop peu de lumière, l'intensité de l'illumination est augmentée. A l'inverse, si le convertisseur lumière/fréquence sature, l'intensité de l'illumination est diminuée. L'illumination en continu et le contrôle de l'intensité d'illumination permet également d'optimiser la qualité du signal reçu par le convertisseur.

Le calcul de la rigidité artérielle d'un patient, humain ou animal, selon l'invention comprend les étapes suivantes :
(a) positionner le premier capteur 100 contre la peau d'un patient située sur une artère carotide ;
(b) positionner le second capteur 200 contre la peau d'un patient située sur une artère fémorale ;

Pour une bonne fiabilité de la mesure, il est nécessaire de positionner les capteurs 100 et 200 du même côté du patient : sur l'artère carotide droite et l'artère fémorale droite, ou sur l'artère carotide gauche et l'artère fémorale gauche. En outre, le patient doit être allongé et le manche du capteur fémorale 200 orienté vers le haut : on insère le capteur sous les vêtements du patient par la partie haute du corps, au-dessus de l'aine.

Le procédé de calcul de la rigidité artérielle selon l'invention comprend également les étapes suivantes :
(c) mesurer la distance (dL) séparant le premier et le deuxième capteur en position d'utilisation ;
(d) actionner les capteurs 100-200 de manière synchrone et simultanée de manière à mesurer l'onde de pouls entre l'artère carotide et l'artère fémorale ;
(g) mesurer la différence de temps entre l'impulsion mesurée au niveau de l'artère carotide et l'impulsion mesurée au niveau de l'artère fémorale ;
(h) calculer la rigidité artérielle comme étant égale à 0,8 x dL/dT, où :
   ▪dL est la distance séparant le premier capteur 100 et le deuxième capteur 200 en position d'utilisation, et
   ▪dT est la différence de temps entre l'impulsion S100 mesurée par le premier capteur 100 au niveau de l'artère carotide et l'impulsion S200 mesurée par le second capteur 200au niveau de l'artère fémorale.

Sur la figure 2, l'enregistrement de l'unité de commande 300 est fait sous la forme d'un graphique du signal de fréquence en fonction du temps.

Avantageusement, l'enregistrement est mémorisé dans une mémoire (non illustrée) afin de pouvoir archiver les enregistrements de chaque patient et pouvoir faire un traitement ultérieur des données afin d'établir, par exemple, l'évolution de la VOP pour chaque patient.

Pour effectuer le calcul de la rigidité artérielle, le dispositif 1 selon l'invention comporte une unité 400 de calcul de la rigidité artérielle, avantageusement intégrée à l'unité de commande 300 et programmée pour :
∘ mesurer la différence de temps dT entre l'impulsion mesurée au niveau de l'artère carotide et l'impulsion mesurée au niveau de l'artère fémorale ; et
∘ calculer la rigidité artérielle comme étant égale à 0,8 x dL/dT, où :
   ▪ dL est la distance séparant le premier capteur 100 et le deuxième capteur 200 en position d'utilisation, et
   ▪ dT est la différence de temps entre l'impulsion mesurée par le premier capteur au niveau de l'artère carotide et l'impulsion mesurée par le second capteur au niveau de l'artère fémorale.

La connaissance de la rigidité artérielle ne suffit pas à poser un diagnostic à des fins curatives. Ce n'est qu'un critère parmi d'autres que le médecin prendra en compte pour poser son diagnostic, après avoir procédé à des examens complémentaires.

La distance dL entre les deux capteurs 100-200 doit être renseignée pour que le dispositif puisse calculer la rigidité artérielle. À cette fin, le dispositif comporte une interface d'entrée de la distance dL séparant le premier et le deuxième capteur en position d'utilisation. Cette interface peut permettre une saisie manuelle de la distance dL par l'opérateur (par exemple un clavier ou une molette), et/ou une saisie automatique par un moyen de mesure automatique de la distance qui sépare les deux capteurs en position d'utilisation (il est possible d'utiliser des capteurs ultrasons ou laser pour mesurer cette distance).

La distance dL entre les deux capteurs 100-200 est la distance directe carotidofémorale qui est retenue pour le calcul de la rigidité artérielle.

Cette distance peut être mesurée manuellement, par exemple à l'aide d'un mètre-ruban ou d'une toise.

Alternativement, l'invention prévoit que les capteurs 100-200 comportent un moyen de mesure de la distance qui les sépare en position d'utilisation. Il peut s'agir, par exemple, de capteur électroniques ou d'une cordelette graduée montée sur enrouleur, de sorte que la distance entre les deux capteurs en position d'utilisation soit immédiatement connue. En particulier, le moyen de mesure est agencé pour indiquer la distance directe entre les deux convertisseurs lumière/fréquence des capteurs 100-200.

Comme illustré sur la figure 2, l'unité de commande comporte un moyen de réglage 310 de la fréquence d'échantillonnage. Selon l'invention, cette fréquence d'échantillonnage est comprise entre 100 mesures par seconde et 1000 mesures par seconde. L'intervalle entre deux mesures est programmé dans l'unité de commande 300 pour effectuer les mesures S100 et S200 sur les capteurs 100 et 200 toutes les millisecondes à toutes les 10 millisecondes. De préférence, la fréquence d'échantillonnage est de 500 mesures par seconde, de sorte que l'intervalle entre deux mesures est de 2 millisecondes. Cette valeur est, selon l'invention, suffisante pour obtenir un signal de bonne qualité. L'unité de commande 300 peut par ailleurs régler de manière automatique l'intensité de chaque source lumineuse 10 des deux capteurs 100 et 200.

Avantageusement, l'unité de calcul 400 est également programmée pour appliquer un filtre passe-bande entre 0,5Hz et 20Hz avant de mesurer la différence de temps dT entre l'impulsion mesurée au niveau de l'artère carotide et l'impulsion mesurée au niveau de l'artère fémorale, et donc avant de calculer la rigidité artérielle.

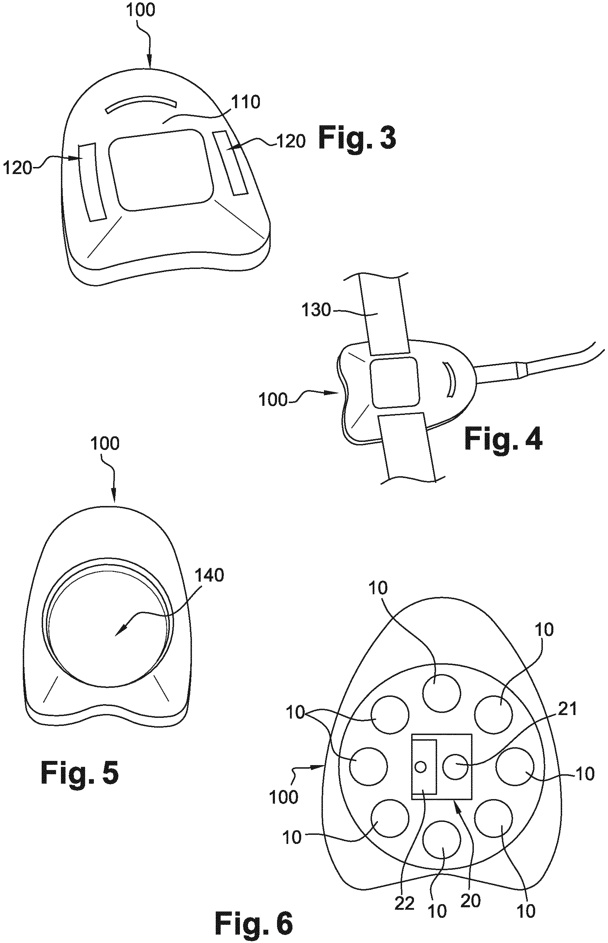

Les figures 3 à 6 illustrent un mode de réalisation du capteur 100 destiné à être positionné sur le cou d'un patient, au-dessus de l'artère carotide.

La figure 3 représente le capteur 100 vu de sa face extérieure, c'est-à-dire celle qui n'est pas en contact avec la peau du patient.

Le capteur comprend un boitier 110 muni de passants 120 pour une sangle 130 (voir figure 4) ou un collier réglable de fixation du capteur autour du cou du patient, en position d'utilisation.

Dans le mode de réalisation de la figure 4, le capteur 100 comporte une prise de branchement pour une alimentation et/ou un câble de transfert des données.

Alternativement, le boitier peut comporter un logement pour une batterie, et un émetteur récepteur radio pour recevoir les commandes et envoyer les données captées.

La figure 5 illustre le boitier 110 vue de dessous, c'est-à-dire vu du côté de la face intérieure destinée à être en contact avec la peau du patient.

Sur cette face intérieure, le boitier présente un évidement 140 pour loger la source lumineuse et le convertisseur lumière/fréquence.

La figure 6 illustre un agencement avantageux de l'invention permettant une mesure efficace et économique.

La source lumineuse est constituée d'une pluralité de diodes électroluminescentes 10, ici huit, et agencées de manière circulaire autour du convertisseur lumière/fréquence 20. Cet agencement permet une illumination suffisante de l'artère grâce au recouvrement des zones d'éclairement des diodes 10 et assure un retour lumineux significatif vers le convertisseur lumière/fréquence 20.

Selon les diodes utilisées, on peut alternativement prévoir un nombre de diodes inférieure, par exemple de deux.

Cet agencement permet une illumination suffisante de l'artère grâce au recouvrement des zones d'éclairement des diodes 10 et assure un retour lumineux significatif vers le convertisseur lumière/fréquence 20.

Le choix des diodes électroluminescentes est important pour obtenir une mesure de bonne qualité et pour déterminer le nombre de diodes nécessaires pour assurer une bonne illumination de l'artère.

Ainsi, selon l'invention et dans cet exemple la source lumineuse de chaque capteur 100-200 est constituée d'une pluralité de diodes électroluminescentes infrarouge émettant à une longueur d'onde de 850 nm.

La qualité du signal reçu est directement liée à la quantité de lumière perçue par le convertisseur lumière/fréquence 20. Pour obtenir des résultats optimaux, chaque source lumineuse est suffisante pour que le convertisseur reçoive une irradiance en µW/cm² comprise entre 20 µW/cm² et 500 µW/cm² après réflexion de la lumière par les artères carotide et fémorale.

Le convertisseur lumière/fréquence 20 comprend une photodiode 21 couplée à un convertisseur courant/fréquence 22.

Lorsque le convertisseur lumière/fréquence 20 est un convertisseur TSL235LF commercialisé par la société Texas Instruments ®, l'irradiance requise est obtenu par l'utilisation d'une source lumineuse composée de huit diodes SFH 4356P commercialisées par la société OSRAM ® et agencées de manière circulaire autour du convertisseur lumière/fréquence.

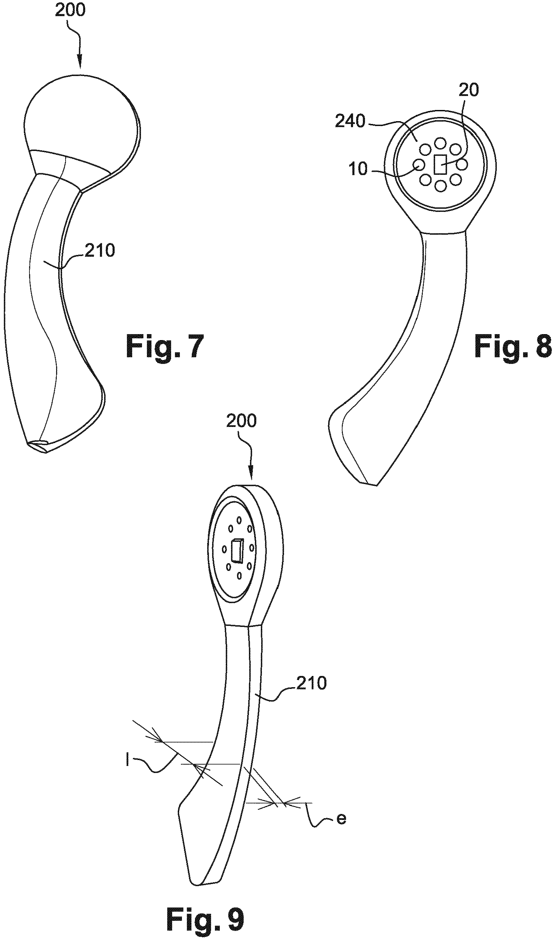

Les figures 7 à 9 illustrent un mode de réalisation du capteur 200 destiné à être positionné au niveau du pli de l'aine du patient, au-dessus de l'artère fémorale.

La difficulté de cette mesure repose sur le fait que le patient est normalement amené à exposer au praticien cette partie du corps. Certaines pratiques ou cultures interdisent une telle exposition. En outre, l'exposition de l'aine peut augmenter le stress du patient et induire une accélération du rythme cardiaque en entraînant une mesure faussée de la VOP.

Selon l'invention, le capteur second 200 est agencé à une extrémité d'un manche 210 de préhension, l'extrémité étant destinée, lorsque le second capteur 200 est placé au niveau de la fémorale, à dépasser de la ceinture du patient. La présence de ce manche 210 permet à l'opérateur de positionner le capteur au-dessus de l'artère fémorale tout en gardant sa main à distance de la zone de l'aine. En outre, le manche permet de glisser le capteur sous les vêtements du patient, lui évitant de découvrir cette zone (voir figure 2). Cela limite le stress du patient et assure une meilleure qualité de mesure et un résultat plus significatif. Cela permet également de proposer une mesure entre les artères carotides et fémorale dans des régions du monde où la rigidité artérielle est habituellement mesurée entre l'artère humérale et l'artère tibiale postérieure.

Cette prise de la mesure à distance au niveau de la fémorale est rendue possible par la technologie utilisée (photopléthysmographie). En effet, la mesure de la vitesse de l'onde de pouls étant obtenue à partir d'une illumination de l'artère, un léger décalage du capteur par rapport à l'artère peut néanmoins permettre d'assurer la mesure, à condition que le signal reçu par le convertisseur soit de qualité suffisante. À cet effet, et comme explicité précédemment, le dispositif de l'invention est réalisé de façon à ce que la qualité de ce signal soit optimum. Différents paramètres sont ajustés à cet effet ; il s'agit notamment de la longueur d'onde de 850nm, de l'illumination en continue, du contrôle de l'intensité d'illumination, de la proximité du convertisseur et des diodes dans les capteurs, ou encore de la fréquence d'échantillonnage qui permettent seuls, et en combinaison, de conférer une bonne qualité du signal en autorisant que le second capteur ne soit pas nécessairement positionné exactement au-dessus de l'artère fémorale.

En référence aux figures 7 à 9, le manche 210 est de forme oblongue et présente une longueur d'au moins 15 centimètres, de préférence une longueur comprise entre 15 et 50 centimètres. Le manche 210 présente en outre une forme sensiblement en arc de cercle, ce qui permet au praticien de maintenir le capteur 200 en position sur la fémorale sans avoir à tenir le manche 210 dans l'axe de l'aine. Le praticien peut ainsi assurer ce maintien avec précision en étant positionné à côté du patient.

Ce manche permet à l'opérateur de positionner le capteur au-dessus de l'artère fémorale tout en gardant sa main à distance de la zone de l'aine. En outre, le manche permet de glisser le capteur sous les vêtements du patient, lui évitant de découvrir cette zone (voir figure 2). Cela limite le stress du patient et assure une meilleure qualité de mesure et un résultat plus significatif.

La figure 7 illustre le capteur 200 vu de sa face extérieure, c'est-à-dire celle qui n'est pas en contact avec la peau du patient.

La figure 8 illustre le capteur 200 vu de sa face intérieure destinée à être en contact avec la peau du patient. L'extrémité du capteur 200 présente un évidement 240 pour loger la source lumineuse et le convertisseur lumière/fréquence.

La figure 9 illustre le capteur 200 vu de trois-quarts. Cette vue montre que le manche 210 est de forme oblongue et avantageusement aplatie, c'est-à-dire qu'il présente une épaisseur h très inférieure à sa largeur l, afin de faciliter son glissement sous les vêtements du patient.

La source lumineuse et le convertisseur sont identiques à ceux du premier capteur, et leur agencement est identique.

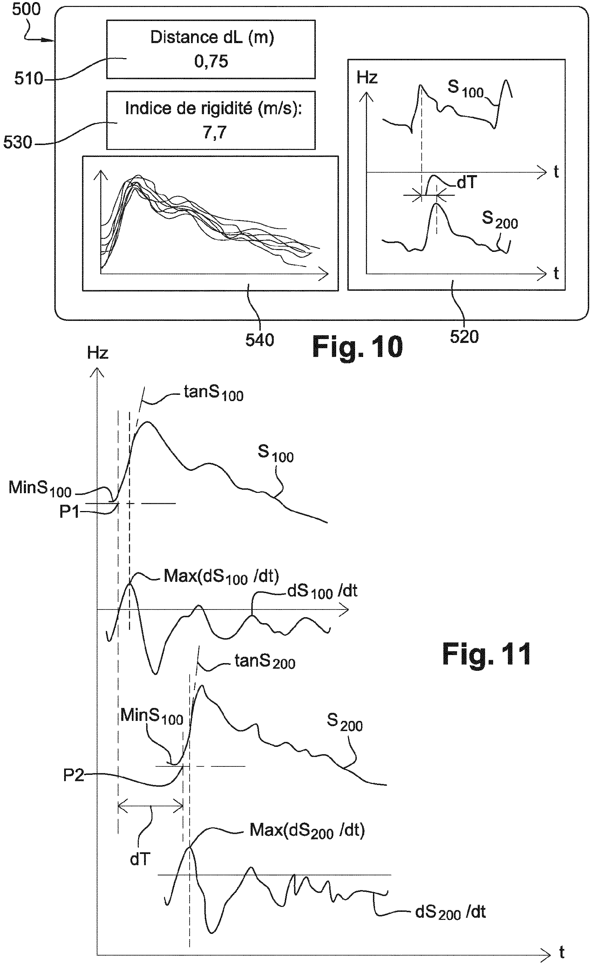

La figure 10 illustre un premier exemple d'interface 500 affichant le signal capté et traité selon une première méthode par le dispositif selon l'invention.

L'interface 500 affiche de préférence :
- dans une première zone 510: la distance dL, mesurée entre le premier capteur 100 positionné au-dessus de l'artère carotide et le second capteur 200 positionné au-dessus de l'artère fémorale. Dans l'exemple : 0,75 mètre ;
- dans une deuxième zone 520 : le signal S100 envoyé par le capteur 100 et le signal S200 envoyé par le capteur 200, ainsi que la méthode de détermination de dT, la différence de temps entre l'impulsion mesurée par le premier capteur au niveau de l'artère carotide et l'impulsion mesurée par le second capteur au niveau de l'artère fémorale ;
- dans une troisième zone 530 : le résultat du calcul de l'indice de rigidité artérielle. Dans l'exemple : 7,7 m/s.

Optionnellement, l'interface peut afficher, dans une quatrième zone 540, le signal brut capté par les convertisseurs 20 des capteurs 100 et 200, afin de permettre à l'opérateur de vérifier que les capteurs sont bien positionnés.

Le dispositif de calcul de la rigidité artérielle selon l'invention peut calculer la différence de temps dT par plusieurs méthodes.

Une première méthode, illustrée en figure 10, et qui donne des résultats corrects est la détection des maxima ou minima des signaux S100 et S200, et de calculer la différence de temps entre ces maxima/minima.

L'invention propose une deuxième méthode permettant d'améliorer la précision du calcul en effectuant un traitement supplémentaire du signal avant le calcul de dT.

Tout d'abord, l'unité de calcul 400 applique un filtre passe-bande entre 0,5Hz et 20Hz sur le signal.

Puis, la mesure de différence de temps dT entre l'impulsion mesurée au niveau de l'artère carotide et l'impulsion mesurée au niveau de l'artère fémorale est effectuée par la méthode des tangentes, tel qu'illustré en figure 11, sur laquelle les signaux issus des capteurs 100-200 sont notés, respectivement, S100 et S200, et les signaux dérivés par rapport au temps sont notés, respectivement, dS100/dt et dS200/dt.

Pour cela, on calcule le maximum Max(dS100/dt) de la dérivée du signal dS100/dt, et on trace la tangente, tanS100, en ce point sur le signal d'origine S100. Ensuite, on relève le point minimum MinS100 sur le signal d'origine et on trace une ligne horizontale tangente en ce point. Le point d'intersection P1 des deux droites est le premier point utilisé pour calculer le temps de transit.

On répète le traitement sur le signal S200 et sa dérivée dS200/dt.

Pour cela, on calcule le maximum Max(dS200/dt) de la dérivée du signal dS200/dt, et on trace la tangente, tanS200, en ce point sur le signal d'origine S200. Ensuite, on relève le point minimum MinS200 sur le signal d'origine et on trace une ligne horizontale tangente en ce point. Le point d'intersection P2 des deux droites est le second point utilisé pour calculer le temps de transit.

Ainsi, la mesure de différence de temps dT entre l'impulsion mesurée au niveau de l'artère carotide et l'impulsion mesurée au niveau de l'artère fémorale est la différence de temps mesurée entre P2 et P1.

Ce mode de réalisation donne des résultats très précis et facilement calculables automatiquement.

L'invention propose donc un dispositif fiable, facile à mettre en oeuvre, permettant une mesure dans la région de l'aine sans générer de stress chez le patient.

## Revendications

1. Dispositif de mesure de la rigidité artérielle d'un patient, humain ou animal, **caractérisé en ce qu'**il comprend :
• un premier capteur (100) destiné à être positionné, en position d'utilisation, contre la peau (P) d'un patient située sur une artère carotide (A), et comprenant au moins une diode électroluminescente formant source lumineuse (10) et émettant à une longueur d'onde comprise entre 600 et 1400 nm, , et un convertisseur lumière/fréquence (20) ;
• un second capteur (200) destiné à être positionné, en position d'utilisation, contre la peau d'un patient située sur une artère fémorale, et comprenant au moins une diode électroluminescente formant source lumineuse (10) et émettant à une longueur d'onde comprise entre 600 et 1400 nm, et un convertisseur lumière/fréquence (20) ; et
• une unité de commande (300) reliée aux capteurs (100-200) par liaison filaire ou hertzienne, adaptée pour :
∘ commander un fonctionnement simultané de la source lumineuse (10) des deux capteurs, et pour
∘ enregistrer des données envoyées par le convertisseur (20) des deux capteurs en réponse à un signal lumineux envoyé par les sources lumineuses (10) et réfléchi respectivement par les artères carotide et fémorale ;
• une unité de calcul (400) de la rigidité artérielle comprenant une interface d'entrée de la distance (dL) séparant le premier capteur (100) et le deuxième capteur (200) en position d'utilisation, et programmée pour :
∘ mesurer la différence de temps (dT) entre l'impulsion (S100) mesurée au niveau de l'artère carotide et l'impulsion (S200) mesurée au niveau de l'artère fémorale
∘ calculer la rigidité artérielle comme étant égale à 0,8 x dL/dT, où :
▪ dL est la distance séparant le premier et le deuxième capteur en position d'utilisation, et
▪ dT est la différence de temps entre l'impulsion mesurée par le premier capteur au niveau de l'artère carotide et l'impulsion mesurée par le second capteur au niveau de l'artère fémorale.

2. Dispositif de mesure de la rigidité artérielle selon la revendication 1, dans lequel l'unité de commande (300) est adaptée pour commander une illumination en continu de la source lumineuse (10) des deux capteurs (100,200), et un ajustement de l'intensité d'illumination de la source lumineuse (10) des deux capteurs (100,200) selon la quantité de lumière reçue par le convertisseur lumière/fréquence (20) concerné.

3. Dispositif de mesure de la rigidité artérielle selon l'une quelconque des revendications 1 et 2, dans lequel chaque source lumineuse est constituée d'une pluralité de diodes électroluminescentes (10), avantageusement agencées autour du convertisseur lumière/fréquence.

4. Dispositif de mesure de la rigidité artérielle selon l'une quelconque des revendications 1 à 3, dans lequel chaque source lumineuse est suffisante pour que le convertisseur reçoive une irradiance en µW/cm² comprise entre 20 µW/cm² et 500 µW/cm² après réflexion de la lumière par les artères carotide et fémorale.

5. Dispositif de mesure de la rigidité artérielle selon l'une quelconque des revendications 1 à 4, dans lequel le premier capteur comporte un collier réglable (130) de fixation du capteur autour du cou du patient, en position d'utilisation.

6. Dispositif de mesure de rigidité artérielle selon l'une quelconque des revendications 1 à 5, dans lequel le second capteur (200) est agencé à une extrémité d'un manche de préhension (210).

7. Dispositif selon la revendication 6, dans lequel le manchon présente une longueur minimum de 15 cm, de préférence une longueur comprise entre 15 et 50 cm.

8. Dispositif de mesure de rigidité artérielle selon l'une quelconque des revendications 1 à 7, dans lequel les capteurs comportent un moyen de mesure de la distance qui les sépare en position d'utilisation.

9. Dispositif de mesure de rigidité artérielle selon l'une quelconque des revendications 1 à 8, les diodes électroluminescentes émettent à une longueur d'onde de 850 nm.

10. Dispositif de mesure de rigidité artérielle selon l'une quelconque des revendications 1 à 9, dans lequel l'unité de calcul (400) comprend un moyen de réglage (310) d'une fréquence d'échantillonnage comprise entre 100 mesures par seconde et 1000 mesures par seconde, de préférence de 500 mesures par seconde.

11. Dispositif de mesure de rigidité artérielle selon l'une quelconque des revendications 1 à 10, dans lequel l'unité de calcul (400) est programmée pour effectuer un filtrage passe-bande entre 0,5Hz et 20Hz avant de mesurer la différence de temps (dT) entre l'impulsion mesurée par le premier capteur au niveau de l'artère carotide et l'impulsion mesurée par le second capteur au niveau de l'artère fémorale.

12. Procédé de calcul de la rigidité artérielle d'un patient, humain ou animal, **caractérisé en ce qu'**il comprend les étapes suivantes :
(a) positionner un premier capteur comprenant au moins une diode électroluminescente formant source lumineuse et émettant à une longueur d'onde comprise entre 600 et 1400 nm, et un convertisseur lumière/fréquence, contre la peau d'un patient située sur une artère carotide ;
(b) positionner un second capteur comprenant au moins une diode électroluminescente formant source lumineuse et émettant à une longueur d'onde comprise entre 600 et 1400 nm, et un convertisseur lumière/fréquence, contre la peau d'un patient située sur une artère fémorale ;
(c) mesurer la distance (dL) séparant le premier et le deuxième capteur en position d'utilisation
(d) actionner les capteurs de manière synchrone et simultanée de manière à mesurer l'onde de pouls entre l'artère carotide et l'artère fémorale ;
(e) mesurer la différence de temps entre l'impulsion mesurée au niveau de l'artère carotide et l'impulsion mesurée au niveau de l'artère fémorale ;
(f) calculer la rigidité artérielle comme étant égale à 0,8 x dL/dT, où :
▪ dL est la distance séparant le premier et le deuxième capteur en position d'utilisation, et
▪ dT est la différence de temps entre l'impulsion mesurée par le premier capteur au niveau de l'artère carotide et l'impulsion mesurée par le second capteur au niveau de l'artère fémorale.

13. Procédé de calcul de la rigidité artérielle selon la revendication 12, comprenant, entre les étapes (d) et (g), les étapes :
(e) définir une fréquence d'échantillonnage comprise entre 100 mesures par seconde et 1000 mesures par seconde, de préférence de 500 mesures par seconde ;
(f) effectuer un filtrage passe-bande entre 0,5Hz et 20Hz.

14. Procédé de calcul de la rigidité artérielle selon la revendication 12 ou 13, dans lequel l'étape (g) de mesure de la différence de temps entre l'impulsion mesurée au niveau de l'artère carotide et l'impulsion mesurée au niveau de l'artère fémorale est effectuée par la méthode des tangentes.

15. Utilisation du dispositif selon l'une quelconque des revendications 1 à 11 pour appréhender un dysfonctionnement cardio-vasculaire.
